Europäisches Patentamt

European Patent Office

Office européen des brevets

(19)

(11) Numéro de publication : **0 106 762**
**B1**

(12)

# FASCICULE DE BREVET EUROPÉEN

(45) Date de publication du fascicule du brevet :
**30.12.86**

(51) Int. Cl.⁴ : **A 61 K 7/032**, A 61 K 7/02

(21) Numéro de dépôt : **83401986.1**

(22) Date de dépôt : **12.10.83**

(54) **Produit cosmétique pour le maquillage.**

(30) Priorité : **12.10.82 FR 8217051**

(43) Date de publication de la demande :
**25.04.84 Bulletin 84/17**

(45) Mention de la délivrance du brevet :
**30.12.86 Bulletin 86/52**

(84) Etats contractants désignés :
**AT BE CH DE FR GB IT LI NL**

(56) Documents cités :
**DE-A- 2 612 330**
**FR-A- 2 076 990**
**FR-A- 2 477 173**
**GB-A- 2 002 652**
**US-A- 3 196 079**
**US-A- 3 936 402**
**CHEMICAL ABSTRACTS, vol. 93, no. 26, décembre**
**1980, page 374, no. 245273q, Columbus, Ohio, US**

(73) Titulaire : **L'OREAL**
**14, Rue Royale**
**F-75008 Paris (FR)**

(72) Inventeur : **Arraudeau, Jean-Pierre**
**48, rue des Moines**
**F-75017-Paris (FR)**
Inventeur : **Patraud, Jeanne**
**142, Bld Masséna**
**F-75013-Paris (FR)**
Inventeur : **Le Gail, Louis**
**15, Placette de la Garrigue Le clos du midi**
**F-91440-Bures S/Yvette (FR)**

(74) Mandataire : **Nony, Michel**
**Conseil en Brevets d'Invention 29 rue Cambacérès**
**F-75008 Paris (FR)**

Jouve, 18, rue St-Denis, 75001 Paris, France

## Description

La présente invention a pour objet un produit cosmétique pour le maquillage du visage.

Les produits de maquillages tels que les fards à paupières, les fards à joues, les anti-cernes, les bâtons à lèvres, etc... permettent d'améliorer l'esthétique en conférant plus de relief au visage, aux yeux et aux lèvres et en intensifiant leur couleur.

Ils peuvent se présenter sous forme aqueuse ou anhydre et sont appliqués sur la surface de la peau des paupières ou des lèvres dans des teintes susceptibles de convenir à chaque utilisatrice.

L'inconvénient rencontré le plus fréquemment lors de l'utilisation des fards à joues et des fards à paupières réside dans le fait qu'après un certain laps de temps, le produit, du fait du plissement de la peau et des paupières, a tendance à perdre de son uniformité et à se concentrer plus ou moins rapidement et profondément dans les plis de la peau. Il en résulte un effet jugé généralement peu esthétique.

Malgré de nombreuses recherches dans ce domaine, il n'a pas été possible de mettre au point des produits de maquillage susceptibles de garder leur uniformité pendant des périodes de temps plus ou moins prolongées c'est-à-dire avoir des propriétés connues sous le terme de « CREASEPROOF ».

La présente invention propose une solution très satisfaisante à ce problème par l'utilisation dans des produits de maquillage d'un nouvel agent liant constitué d'un mélange intime de silice et de fibres de polyéthylène.

Les études effectuées ont en effet permis de montrer que les produits de maquillage contenant cet agent liant ne provoquaient pas la formation de plis inesthétiques, empêchaient la migration du maquillage et ne nécessitaient pas, dès lors, de nouvelles applications comme ceci est le plus souvent le cas avec les produits conventionnels.

L'utilisation de polyéthylène, notamment sous forme de poudre, dans des compositions cosmétiques, a déjà fait l'objet de nombreux brevets parmi lesquels on peut citer les brevets U.S. 3.196.079 et 3.936.402, le brevet britannique 2.002.652 et le brevet français 71.02485.

La présente invention a pour objet l'utilisation en tant qu'agent liant, d'un mélange intime constitué de 5 à 95 % en poids de silice finement subdivisée et de 95 à 5 % en poids de fibres de polyéthylène finement subdivisées ayant une structure fibrillaire pour la préparation d'un produit destiné au maquillage du visage.

L'agent liant est de préférence obtenu par le broyage à énergie fluide de silice et de polyéthylène par exemple à l'aide d'un microniseur.

Le cobroyage des fibres de polyéthylène et de la silice diminue la dimension de particule de la silice, défibrille les fibres de polyéthylène et produit un mélange intime, les fibres de polyéthylène et les particules de silice étant maintenues ensemble et ne pouvant être séparées par un moyen mécanique normal.

Le polyéthylène du mélange intime a un poids moléculaire supérieur à environ 400 000, un point de ramollissement de l'ordre de 120-130 °C et un point de fusion de l'ordre de 130 à environ 135 °C. Le polyéthylène finement subdivisé contient une quantité supérieure à environ 90 % en poids de fibres ayant une longueur moyenne de l'ordre d'environ 10 μm et un diamètre de moins d'environ 1 μm. De préférence les fibres de polyoléfine sont très fibrillées et ont une aire spécifique très élevée généralement comprise entre 5 et 15 m²/g.

La silice est de préférence un aérogel de silice (silice amorphe synthétique) ayant un diamètre moyen des particules en poids de l'ordre de 2 à environ 10 μ, une aire superficielle de l'ordre de 300 à environ 400 m²/g, un volume de pores d'au moins environ 1,2 cm³/g et un diamètre moyen des pores de l'ordre de 150 à environ 250 Å.

Selon un mode particulier de réalisation le mélange intime contient entre environ 45 et environ 75 % et de préférence entre 50 et 60 % en poids d'aérogel de silice ayant un diamètre moyen en poids des particules de l'ordre de 2 à 10 μm et entre environ 55 et 25 % et de préférence entre 40 et 50 % en poids de fibres de polyéthylène finement subdivisée d'aire spécifique comprise entre 5 et 15 m²/g.

L'agent liant, tel que défini ci-dessus, peut être l'un de ceux décrits dans le brevet français 81.03535, notamment les produits vendus sous les marques de commerce de « SP 5-1776 »®, (60 % de silice amorphe — 40 % de polyéthylène basse densité d'aire spécifique de 6 à 10 m²/g et de « SP 5-2076 »® (50 % de silice amorphe — 50 % de polyéthylène basse densité d'aire spécifique de 6 à 10 m²/g).

L'agent liant est généralement présent en une proportion comprise entre 0,5 et 20 % en poids.

Les produits cosmétiques destinés au maquillage peuvent se présenter soit sous forme anhydre, soit sous forme aqueuse. Dans ce dernier cas il s'agit plus particulièrement d'une émulsion du type huile-dans-l'eau ou eau-dans-l'huile ou encore d'une suspension.

Lorsqu'ils se présentent sous forme anhydre il peut s'agir soit d'une poudre compactée ou non soit d'un produit contenant des corps gras et éventuellement des solvants organiques.

Lorsque les produits selon l'invention se présentent sous forme de poudre, compactée ou non, ils sont généralement constitués :

de 0,5 à 20 % de l'agent liant
de 0 à 20 % d'un corps gras

de 1 à 70 % de pigment(s) coloré(s)
et de 5 à 90 % d'une charge minérale ou organique telle que du talc et de l'amidon.

Lorsque les produits selon l'invention se présentent sous forme grasse anhydre ils sont généralement constitués :

de 0,5 à 20 % de l'agent liant
de 5 à 98 % d'un corps gras
de 0 à 80 % d'un solvant
et de 1 à 25 % de pigment(s) coloré(s)

Selon cette forme de réalisation les produits de maquillage peuvent également contenir des charges minérales ou organiques.

Le corps gras est au môins une huile ou un mélange d'au moins une huile et d'au moins une cire.

Parmi les huiles susceptibles d'être utilisées selon l'invention, on peut en particulier citer :

parmi les huiles minérales : l'huile de paraffine, l'huile de vaseline et les huiles minérales ayant un point d'ébullition compris entre 310 et 410 °C,

parmi les huiles d'origine animale : l'huile de Purcellin, le perhydrosqualène et l'huile de calophyllum, parmi les huiles végétales : l'huile d'amande douce, l'huile de palme, l'huile d'avocat, l'huile d'olive, l'huile de ricin, les huiles de germes de céréales telles que l'huile de germes de blé,

parmi les huiles de silicone : le diméthylpolysiloxane,

parmi les esters de synthèse : le myristate de butyle, le myristate d'isopropyle, le myristate de cétyle, le palmitate d'isopropyle, le stéarate de butyle, le stéarate d'hexadécyle, le stéarate d'isopropyle, le stéarate d'octyle, le stéarate d'isocétyle, l'oléate de décyle, le laurate d'hexyle, le dicaprylate de propylène glycol, l'adipate de di-isopropyle,

parmi les alcools organiques : l'alcool oléique, l'alcool linoléique, l'alcool linolénique, l'alcool isostéarylique, l'octyl dodécanol,

parmi les esters dérivés d'acide lanolique : le lanolate d'isopropyle, le lanolate d'isocétyle,

parmi les huiles on peut également citer : les acétyl-glycérides, les octanoates et décanoates d'alcools et de polyalcools tels que ceux de glycol et glycérol, les ricinoléates d'alcools et de polyalcools tels que celui de cétyle.

Parmi les cires susceptibles d'être utilisées selon l'invention, on peut mentionner :

parmi les cires minérales : les cires microcristallines, la paraffine, la vaseline,

parmi les cires fossiles : l'ozokérite, la cire de montan,

parmi les cires d'origine animale : la cire d'abeille, le spermaceti, la cire de lanoline, les dérivés issus de la lanoline tels que les alcools de lanoline, la lanoline hydrogénée, la lanoline hydroxylée, la lanoline acétylée, les acides gras de la lanoline, l'alcool de lanoline acétylé,

parmi les cires d'origine végétale : la cire de candellila, la cire de Carnauba, la cire du Japon, le beurre de cacao,

parmi les huiles hydrogénées concrètes à 25 °C : l'huile de ricin hydrogénée, l'huile de palme hydrogénée, le suif hydrogéné, l'huile de coco hydrogénée,

parmi les cires synthétiques : les cires de polyéthylène, les cires de polyéthylène copolymérisées, parmi les esters gras concrets à 25 °C : le monomyristate de propylène glycol, le myristate de myristyle,

parmi les cires de silicone : le méthyloctadécane-oxypolysiloxane et le poly(diméthylsiloxy) stéaroxy-siloxane,

parmi les cires on peut également citer : l'alcool cétylique, l'alcool stéarylique, les mono, di et triglycérides concrets à 25 °C, la monoéthanolamide stéarique, la colophane et ses dérivés tels que les abiétates de glycol et de glycérol, les sucro glycérides et les oléates, myristates, lanolates, stéarates et dihydroxystéarates de calcium, magnésium, zinc et aluminium.

Parmi les solvants susceptibles d'être utilisés on peut en particulier mentionner : les isoparaffines, les silicones cycliques ou linéaires à point d'ébullition inférieur à 200 °C et les solvants chlorés.

Comme pigments colorés on peut mentionner le noir de carbone ou l'oxyde de fer noir, les oxydes de chrome, les oxydes de fer jaune, brun, et rouge, les outre-mers (polysulfures d'aluminosilicates), le pyrophosphate de manganèse, le bleu ferrique, le bioxyde de titane et enfin certaines poudres métalliques telles que celles d'argent ou d'aluminium. Les pigments sont le plus souvent utilisés en mélange avec des agents nacrants tels que l'oxychlorure de bismuth, le micatitane et les cristaux de guanine et certains colorants organiques tels que le carmin de cochenille et les laques organiques.

En outre, ces compositions peuvent également contenir des agents anti-oxydants tels que les esters propylique, octylique et dodécylique de l'acide gallique, l'hydroxy-anisole butylé, l'hydroxy-toluène butylé et l'acide nordihydroguaiarétique ainsi que des parfums, des agents conservateurs ou des agents épaississants tels que les dérivés de la cellulose, les gommes de xanthane, les gommes de guart, les gommes de caroube, les alginates et carreghénates, les complexes silicatés et les argiles modifiées organiquement.

Les émulsions lorsqu'elles se présentent sous forme eau-dans-l'huile sont constituées :

de 5 à 80 % d'une phase eau
de 1 à 40 % d'une phase huile
de 2 à 15 % d'un agent émulsionnant
de 0,5 à 20 % de l'agent liant
de 0 à 20 % d'agent(s) épaississant(s)
et de 0 à 30 % et de préférence de 1 à 25 % de pigment(s) coloré(s).

Les émulsions lorsqu'elles se présentent sous forme huile-dans-l'eau sont constituées :

de 1 à 96,5 % d'une phase eau
de 1 à 30 % d'une phase huile
de 1 à 15 % d'un agent émulsionnant
de 0,5 à 20 % de l'agent liant
de 0 à 20 % d'agent(s) épaississant(s)
et de 0 à 30 % et de préférence de 1 à 25 % de pigment(s) coloré(s).

Pour constituer la phase huile des émulsions on peut utiliser au moins une huile telle qu'énumérée ci-dessus éventuellement en mélange avec au moins une cire.

Parmi les agents émulsionnants susceptibles de convenir aux émulsions on peut en particulier mentionner : les tensio-actifs anioniques et en particulier les savons d'amines ou les sels de l'acide lanolique et les tensio-actifs non-ioniques.

Les pigments employés dans les émulsions sont identiques à ceux énumérés ci-dessus pour les produits anhydres.

Les produits de maquillage selon l'invention peuvent également contenir divers ingrédients tels que des stérols, de la lécithine, des polyalcools, des agents anti-oxydants, des parfums, des agents conservateurs, des charges telles que du talc, de l'amidon de riz ou de blé, de la poudre d'avoine ou de germes de blé, du kaolin ainsi que certains produits actifs.

Comme composés actifs on peut citer notamment des kératolytiques, des anti-inflammatoires, des cicatrisants, le peroxyde de benzyle, l'acide salicylique, l'acide rétinoïque et le collagène.

On va maintenant donner à titre d'illustration et sans aucun caractère limitatif plusieurs exemples de produits de maquillage selon l'invention.

### Exemple 1

Fard à paupières anhydre

| | |
|---|---|
| dicaprylate de propylène glycol | 27,3 g |
| vaseline | 9,1 g |
| huile de vaseline | 27,3 g |
| monostéarate de propylène glycol | 13,6 g |
| trilaurine | 9,1 g |
| cire microcristalline | 4,5 g |
| parahydroxybenzoate de propyle | 0,2 g |
| butyl hydroxy toluène | 0,1 g |
| agent liant « SP 5-1776 »® vendu par la Société W. R. GRACE & Co (polyéthylène + silice) | 1,8 g |
| mica-titane | |
| bioxyde de titane | 7 g |
| ferrocyanure ferrique | |
| | 100 g |

### Exemple 2

Fard à paupières anhydre

| | |
|---|---|
| cire d'abeille | 15 g |
| vaseline | 5 g |
| huile de ricin hydrogénée | 5 g |
| cire de polyéthylène | 10 g |
| isoparaffine | 44,8 g |
| parahydroxybenzoate de propyle | 0,2 g |
| talc | 5 g |
| agent liant « SP 5-1776 »® vendu par la Société W. R. GRACE & Co (polyéthylène + silice) | 2 g |

mica-titane
bioxyde de titane                                                              13   g
oxyde de fer

                                                                              ─────────
                                                                              100   g


## Exemple 3

Fard à paupières sous forme d'une émulsion eau-dans-l'huile

| | |
|---|---|
| ester d'acide gras et de sorbitan | 4 g |
| cire microcristalline | 5 g |
| cire d'abeille | 2 g |
| huile de paraffine | 8 g |
| parahydroxybenzoate de méthyle | 0,3 g |
| agent liant « SP 5-2076 »® vendu par la société W. R. GRACE & Co (polyéthylène + silice) | 5 g |
| mica-titane | |
| bioxyde de titane | 10 g |
| eau désionisée q.s.p. | 100 g |

## Exemple 4

Fard à paupières sous forme d'une émulsion huile-dans-l'eau

| | |
|---|---|
| stéarate de propylène glycol | 3 g |
| acide stéarique | 1 g |
| triéthanolamine | 0,5 g |
| butylène glycol | 10 g |
| méthyl hydroxypropyl cellulose | 0,5 g |
| parahydroxybenzoate de méthyle | 0,15 g |
| parahydroxybenzoate de propyle | 0,15 g |
| mica-titane | |
| bioxyde de titane | 10 g |
| oxyde de chrome anhydre | 2 g |
| bleu outremer | 1 g |
| agent liant « SP 5-2076 »® vendu par la Société W. R. GRACE & Co (polyéthylène + silice) | 10 g |
| eau désionisée q.s.p. | 100 g |

## Exemple 5

Fard à paupières sous forme de poudre

| | |
|---|---|
| talc | 48,74 g |
| colorants : bleu outremer | 6 g |
| oxyde de fer | 3 g |
| oxyde de chrome | 5 g |
| myristate d'isopropyle | 1 g |
| phytostérol | 1 g |
| huile de ricin | 1,7 g |
| huile de vaseline | 9 g |
| alcool oléique | 1,5 g |
| huile protéinée | 1 g |
| anti-oxydants | 0,06 g |
| agent liant « SP 5-1776 »® | 2 g |
| agents nacrants (micatitane) | 20 g |

## Exemple 6

Anti-cernes sous forme d'une émulsion eau-dans-l'huile

| | |
|---|---|
| Ester de sorbitan et d'acide gras | 4 g |
| Palmitate d'isopropyle | 10 g |
| Myristate d'isopropyle | 10 g |
| Cire d'abeille | 4 g |
| Cire de Carnauba | 1 g |

| | |
|---|---|
| Imidazolidinyl urée | 0,3 g |
| Parhydroxybenzoate de méthyle | 0,1 g |
| Collagène soluble | 1 g |
| Oxydes de fer | 5 g |
| Dioxyde de titane | 5 g |
| Mica-titane | 5 g |
| Agent liant « SP 5-2076 »® vendu par la société W. R. GRACE & Co (silice + polyéthylène) | 1 g |
| Eau permutée stérile q.s.p. | 100 g |

Exemple 7

Fard à joues anhydre en stick

| | |
|---|---|
| Huile de paraffine | 50,65 g |
| Vaseline | 10 g |
| Cire de Carnauba | 15 g |
| Ozokérite | 5 g |
| Parhydroxybenzoate de propyle | 0,1 g |
| Butyl Hydroxy Toluène | 0,05 g |
| D C Red 7 | 0,2 g |
| Oxydes de fer | 2 g |
| Dioxyde de titane | 5 g |
| Mica titane | 10 g |
| Agent liant « SP 5-1776 »® | 2 g |

Exemple 8

Camoufleur pour le visage sous forme anhydre

| | |
|---|---|
| Cire de candellila | 4 g |
| Cire microcristalline | 8 g |
| Beurre de Cacao | 8 g |
| Myristate d'isopropyle | 44,9 g |
| Talc | 7 g |
| Butyl Hydroxy Toluène | 0,1 g |
| Oxydes de fer | 5 g |
| Dioxyde de titane | 20 g |
| Agent liant « SP 5-2076 »® | 3 g |

Exemple 9

Anticernes sous forme d'une émulsion huile-dans-l'eau

| | |
|---|---|
| Huile de Vaseline | 26 g |
| Cire d'Abeille | 10 g |
| Borate de Sodium | 1 g |
| Parahydroxybenzoate de propyle | 0,15 g |
| Imidazolidinyl Urée | 0,30 g |
| Oxydes de fer | 5 g |
| Dioxyde de Titane | 5 g |
| Agent liant « SP 5-1776 » ® | 1 g |
| Eau permutée stérile q.s.q. | 100 g |

Exemple 10

Fards à joues sous forme d'une émulsion eau-dans-l'huile

| | |
|---|---|
| Lanolate de Magnésium | 4 g |
| Lanoline Hydrogénée | 3 g |
| Huile de Paraffine | 17 g |
| Cire Microcristalline | 3 g |
| Glycérides d'acides gras saturés | 5 g |
| Parahydroxybenzoate de Méthyle | 0,3 g |
| Oxydes de fer | 2,5 g |
| Dioxyde de Titane | 5 g |

| | |
|---|---|
| Agent liant « SP 5-2076 » ® | 0,5 g |
| Eau permutée stérile q.s.p. | 100 g |

## Exemple 11

Fards à joues sous forme d'une émulsion huile-dans-l'eau

| | |
|---|---|
| Acide Stéarique | 2,5 g |
| Monostéarate de Sorbitan Polyéthoxylé | 0,5 g |
| Huile de Vaseline | 10 g |
| Myristate d'isopropyle | 10 g |
| Triéthanolamine | 1 g |
| Sorbitol | 5 g |
| Paraffine | 1 g |
| Cire de Carnauba | 2 g |
| Silicate de Magnésium | 3 g |
| Oxyde de Fer Rouge | 5 g |
| Dioxyde de Titane | 2 g |
| Mica-Titane | 3 g |
| Imidazolidinyl Urée | 0,3 g |
| Agent liant « SP 5-1776 » ® | 1 g |
| Eau permutée stérile q.s.p. | 100 g |

## Exemple 12

Stick pour les lèvres

| | |
|---|---|
| Cire d'abeille | 8 g |
| Dicaprylate d'éthylène glycol | 16,5 g |
| Paraffine | 3 g |
| Cire de Carnauba | 5 g |
| Trilaurine | 10 g |
| Huile de ricin hydrogénée | 5 g |
| Butyl hydroxy toluène | 0,1 g |
| Lanoline | 1 g |
| Kaolin | 5 g |
| Amidon modifié | 5 g |
| Agent liant « SP 5-1776 » ® | 1 g |
| Isoparaffine | 40,4 g |
| | 100,0 g |

## Exemple 13

Stick pour les lèvres

| | |
|---|---|
| Cire d'abeille | 15 g |
| Vaseline | 12 g |
| Cire de polyéthylène | 10 g |
| Trilaurine | 10 g |
| Huile de ricin hydrogénée | 3 g |
| Lanoline liquide | 9 g |
| Butyl hydroxy toluène | 0,1 g |
| Talc micronisé | 3 g |
| Amidon modifié | 5 g |
| Agent liant « SP 5-1776 » ® | 0,5 g |
| Silicone volatil | 32,4 g |
| | 100,0 g |

Les sticks des exemples 12 et 13 appliqués préalablement à un rouge à lèvres permettent de prévenir la migration du rouge sur les bords des lèvres et assurent ainsi un contour net des lèvres dans le temps.

**Revendications**

1. Utilisation, en tant qu'agent liant, d'un mélange intime constitué de 5 à 95 % en poids de silice finement subdivisée et de 95 à 5 % en poids de fibres de polyéthylène finement subdivisées ayant une structure fibrillaire pour la préparation d'un produit cosmétique destiné au maquillage du visage.

2. Utilisation, selon la revendication 1, caractérisée par le fait que le mélange intime est constitué de 45 à 75 % en poids d'aérogel de silice (silice amorphe) ayant un diamètre moyen en poids de particules de l'ordre de 2 à 10 μm et de 55 à 25 % en poids de fibres de polyéthylène finement subdivisées d'aire spécifique comprise entre 5 et 15 m²/g.

3. Utilisation selon l'une quelconque des revendications 1 et 2, caractérisée par le fait que le mélange intime constitué de 50 à 60 % de silice amorphe et de 40 à 50 % de polyéthylène basse densité d'aire spécifique de 6 à 10 m²/g.

4. Utilisation selon l'une quelconque des revendications précédentes, caractérisée par le fait que le mélange intime constituant l'agent liant est employé en une proportion comprise entre 0,5 et 20 % en poids.

5. Produit cosmétique pour le maquillage, caractérisé par le fait qu'il se présente sous forme d'une poudre compactée ou non et est constitué de :

0,5 à 20 % d'un agent liant tel que défini à l'une quelconque des revendications 1 à 4,
0 à 20 % d'un corps gras,
1 à 70 % de pigment(s) coloré(s),
et 5 à 90 % d'une charge minérale ou organique.

6. Produit cosmétique pour le maquillage caractérisé par le fait qu'il se présente sous forme grasse anhydre et est constitué de :

0,5 à 20 % d'un agent liant tel que défini à l'une quelconque des revendications 1 à 4,
5 à 98 % d'un corps gras,
0 à 80 % d'un solvant,
et 1 à 25 % de pigment(s) coloré(s).

7. Produit selon la revendication 6, caractérisé par le fait que le solvant est pris dans le groupe constitué par : l'isoparaffine, les silicones cycliques ou linéaires ayant un point d'ébullition inférieur à 200 °C et les solvants chlorés.

8. Produit cosmétique pour le maquillage sous forme d'une émulsion constituée d'une phase eau, d'une phase huile, d'un agent émulsionnant et d'un agent liant, caractérisé par le fait que ledit agent liant est présent à raison de 0,5 à 20 % en poids et est tel que défini à l'une quelconque des revendications 1 à 4.

9. Produit selon la revendication 8, caractérisé par le fait que l'émulsion est sous forme eau-dans-l'huile et est constituée de :

5 à 80 % de la phase eau,
1 à 40 % de la phase huile,
2 à 15 % de l'agent émulsionnant,
0,5 à 20 % de l'agent liant,
0 à 20 % d'au moins un agent épaississant,
et 0 à 30 % et de préférence 1 à 25 % de pigment(s) coloré(s).

10. Produit selon la revendication 8, caractérisé par le fait que l'émulsion est sous forme huile-dans-l'eau et est constitué de :

1 à 96,5 % de la phase eau,
1 à 30 % de la phase huile,
1 à 15 % de l'agent émulsionnant,
0,5 à 20 % de l'agent liant,
0 à 20 % d'au moins un agent épaississant,
et 0 à 30 % et de préférence 1 à 25 % de pigment(s) coloré(s).

11. Produit selon l'une quelconque des revendications 5 à 10, caractérisé par le fait que les pigments colorés sont pris dans le groupe constitué par l'oxyde de fer noir, le noir de carbone, les oxydes de chrome, les oxydes de fer jaune, brun et rouge, les outremers, le bioxyde de titane et les poudres métalliques d'argent et d'aluminium, le pyrophosphate de manganèse, le bleu ferrique, le carmin de cochenille et les laques organiques.

12. Produit selon l'une quelconque des revendications 5 à 11, caractérisé par le fait que les pigments colorés sont utilisés en mélange avec des agents nacrants pris dans le groupe constitué par l'oxychlorure de bismuth, le mica-titane et les cristaux de guanine.

8

**Claims**

1. Use, as a binding agent, of an intimate mixture consisting of 5 to 95 % by weight of very finely divided silica and 95 to 5 % by weight of very finely divided polyethylene fibres having a fibrillar structure, for the preparation of a cosmetic product intended for making up the face.

2. Use according to Claim 1, characterized in that the intimate mixture consists of 45 to 75 % by weight of silica aerogel (amorphus silica) having a weight average particle diameter of the order of 2 to 10 $\mu$m and 55 to 25 % by weight of very finely divided polyethylene fibres having a specific surface area of between 5 and 15 $m^2/g$.

3. Use according to either of Claims 1 and 2, characterized in that the intimate mixture consists of 50 to 60 % of amorphous silica and 40 to 50 % of low density polyethylene having a specific surface area of 6 to 10 $m^2/g$.

4. Use according to any one of the preceding claims, characterized in that the intimate mixture constituting the binding agent is used in a proportion of between 0.5 and 20 % by weight.

5. Cosmetic product for make-up, characterized in that it takes the form of a compacted or non-compacted powder and consists of :

0.5 to 20 % of a binding agent as defined in any one of Claims 1 to 4,
0 to 20 % of a fatty substance,
1 to 70 % of coloured pigment(s), and
5 to 90 % of a inorganic or organic filler.

6. Cosmetic product for make-up, characterized in that it takes the form of an anhydrous grease and consists of :

0.5 to 20 % of a binding agent as defined in any one of Claims 1 to 4,
5 to 98 % of a fatty substance,
0 to 80 % of a solvent, and
1 to 25 % of coloured pigment(s).

7. Product according to Claim 6, characterized in that the solvent is chosen from the group consisting of : isoparaffin, cyclic or linear silicones having a boiling point of below 200 °C and chlorinated solvents.

8. Cosmetic product for make-up, in the form of an emulsion consisting of a water phase, an oil phase, an emulsifying agent and a binding agent, characterized in that the said binding agent is present in the proportion 0.5 to 20 % by weight and is a defined in any one of Claims 1 to 4.

9. Product according to Claim 8, characterized in that the emulsion is in water-in-oil form and consists of :

5 to 80 % of the water phase,
1 to 40 % of the oil phase,
2 to 15 % of the emulsifying agent,
0.5 to 20 % of the binding agent,
0 to 20 % of at least one thickening agent, and
0 to 30 %, and preferably 1 to 25 %, of coloured pigment(s).

10. Product according to Claim 8, characterized in that the emulsion is in oil-in-water form and consists of :

1 to 96.5 % of the water phase,
1 to 30 % of the oil phase,
1 to 15 % of the emulsifying agent,
0.5 to 20 % of the binding agent,
0 to 20 % of at least one thickening agent, and
0 to 30 %, and preferably 1 to 25 %, and preferably 1 to 25 %, of coloured pigment(s).

11. Product according to any one of Claims 5 to 10, characterized in that the coloured pigments are chosen from the group consisting of black iron oxide, carbon black, chromium oxides, yellow, brown and red iron oxides, ultramarines, titanium dioxide and powdered metallic silver and aluminium, manganese pyrophosphate, ferric blue, cochineal carmine and organic lakes.

12. Product according to any one of Claims 5 to 11, characterized in that the coloured pigments are used mixed with pearlescent agents chosen from the group consisting of bismuth oxychloride, titanium-mica and guanine crystals.

**Patentansprüche**

1. Verwendung einer innigen Mischung, die aus 5 bis 95 Gew.-% feinverteilten Siliciumdioxid und 95 bis 5 Gew.-% feinverteilten Polyethylenfasern mit fibrillärer Struktur besteht, als Bindemittel zur Herstellung eines kosmetischen Produktes zum Schminken des Gesichts.

2. Verwendung nach Anspruch 1, dadurch gekennzeichnet, daß die innige Mischung aus 45 bis 75 Gew.-% Siliciumdioxidaerogel (amorphes Siliciumdioxid) mit einem gewichtsdurchschnittlichen Teilchendurchmesser von 2 bis 10 μm und aus 55 bis 25 Gew.-% feinverteilten Polyethylenfasern mit einer spezifischen Oberfläche von 5 bis 15 m²/g besteht.

3. Verwendung nach einem der Ansprüche 1 oder 2, dadurch gekennzeichnet, daß die innige Mischung aus 50 bis 60 % amorphem Siliciumoxid und 40 bis 50 % Polyethylen niedriger Dichte mit einer spezifischen Oberfläche von 6 bis 10 m²/g besteht.

4. Verwendung nach einem der vorhergehenden Ansprüche, dadurch gekennzeichnet, daß die innige Mischung welche das Bindemittel darstellt, in einem Anteil von 0,5 bis 20 Gew.-% eingesetzt wird.

5. Kosmetisches Schminkprodukt, dadurch gekennzeichnet, daß es in Form eines gegebenenfalls kompaktierten Pulvers vorliegt und aus

0,5 bis 20 % eines Bindemittels gemäß einem der Ansprüche 1 bis 4,
0-20 % Fettkörper,
1-70 % Farbpigment(en), und
5-90 % eines mineralischen oder organischen Füllstoffs

besteht.

6. Kosmetisches Schminkprodukt, dadurch gekennzeichnet, daß es in wasserfreier, fettartiger Form vorliegt und aus

0,5-20 % eines Bindemittels gemäß einem der Ansprüche 1 bis 4,
5-98 % Fettkörper,
0-80 % Lösungsmittel, und
1-25 % Farbpigment(en)

besteht.

7. Produkt nach Anspruch 6, dadurch gekennzeichnet, daß das Lösungsmittel ausgewählt ist aus der Gruppe bestehend aus Isoparaffin, cyclischen oder linearen Silikonen mit einem Siedepunkt unterhalb 200 °C und chlorierten Lösungsmitteln.

8. Kosmetisches Schminkprodukt in Form einer Emulsion, die aus einer Wasserphase, einer Ölphase, einem Emulgator und einem Bindemittel besteht, dadurch gekennzeichnet, daß das Bindemittel in einem Anteil von 0,2 bis 20 Gew.-% vorhanden ist und daß es sich bei dem Bindemittel um ein Bindemittel gemäß einem der Ansprüche 1 bis 4 handelt.

9. Produkt nach Anspruch 8, dadurch gekennzeichnet, daß die Emulsion eine Wasser-in-Öl-Emulsion ist und aus

5-80 % Wasserphase
1-40 % Ölphase
2-15 % Emulgator
0,5-20 % Bindemittel
0-20 % mindestens eines Verdickungsmittels und
0-30 % und vorzugsweise 1-25 % Farbpigment(en)

besteht.

10. Produkt nach Anspruch 8, dadurch gekennzeichnet, daß die Emulsion eine Öl-in-Wasser-Emulsion ist und aus

1-96,5 % Wasserphase
1-30 % Ölphase
1-15 % Emulgator
0,5-20 % Bindemittel
0-20 % mindestens eines Verdickungsmittels und
0-30 % und vorzugsweise 1-25 % Farbpigment(en)

besteht.

11. Produkt nach einem der Ansprüche 5 bis 10, dadurch gekennzeichnet, daß die Farbpigmente ausgewählt sind aus der Gruppe bestehend aus : Schwarzem Eisenoxid ; Rußschwarz ; Chromoxiden ;

**0 106 762**

gelben, braunen und roten Eisenoxiden, Ultramarinen, Titandioxid und metallischen Silber- und Aluminiumpulvern, Magnesiumpyrophosphat, Eisenblau, Cochenillekarmin und organischen Lacken.

12. Produkt nach einem der Ansprüche 5 bis 11, dadurch gekennzeichnet, daß die Farbpigmente in Mischung mit Perlmuttschimmer verleihenden Mitteln verwendet werden, welche ausgewählt sind aus der Gruppe bestehend aus Wismutoxychlorid, Titanglimmer und Guaninkristallen.